(19) Europäisches Patentamt
European Patent Office
Office européen des brevets

(11) **EP 4 631 425 A1**

(12) **EUROPEAN PATENT APPLICATION**

(43) Date of publication:
**15.10.2025 Bulletin 2025/42**

(21) Application number: **24168931.4**

(22) Date of filing: **08.04.2024**

(51) International Patent Classification (IPC):
*A61B 5/055* (2006.01)   *A61B 5/06* (2006.01)
*G01R 33/28* (2006.01)   *G01R 33/26* (2006.01)
*G01R 33/567* (2006.01)   *G01R 33/36* (2006.01)
*A61B 5/00* (2006.01)

(52) Cooperative Patent Classification (CPC):
**G01R 33/287; A61B 5/055; A61B 5/062;
A61B 5/064; G01R 33/3692; G01R 33/56509;**
G01R 33/5673

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.
5656 AG Eindhoven (NL)**

(72) Inventors:
• **STEHNING, Christian**
  **Eindhoven (NL)**
• **RAHMER, Juergen Erwin**
  **Eindhoven (NL)**
• **NIELSEN, Tim**
  **5656AG Eindhoven (NL)**
• **GLEICH, Bernhard**
  **Eindhoven (NL)**

(74) Representative: **Philips Intellectual Property &
Standards
High Tech Campus 52
5656 AG Eindhoven (NL)**

(54) **MAGNETIC RESONANCE BASED DEVICE LOCALIZATION, ACTUATION AND COMMUNICATION**

(57)    Method and system for localization of a marker in a magnetic resonance imaging system are disclosed. The method comprises: generating during a magnetic resonance imaging sequence at least two radiofrequency pulses, wherein at least one is associated to magnetic resonance imaging and at least one that is associated to localization of the marker in a magnetic resonance imaging field, and which is distinct from the at least one radiofrequency pulse associated to magnetic resonance imaging; generating, by the at least one radiofrequency pulse associated to localization of the marker, a signal receivable during data acquisition of the of the magnetic resonance imaging sequence; receiving data from the magnetic resonance imaging field during the duration of data acquisition period within the magnetic resonance imaging sequence; deriving magnetic resonance imaging data and marker localization data from the received data; outputting the magnetic resonance imaging data and the marker localization data for the magnetic resonance imaging field. The system comprises a computational system configured to perform the method.

Fig. 6

EP 4 631 425 A1

## Description

## TECHNICAL FIELD

The subject-matter described herein relates to systems and methods for device localization and position tracking in magnetic resonance imaging.

## BACKGROUND

Diagnosis and treatment of several pathologies require additional measurements that cannot be provided by imaging alone. Examples include the localized measurement of pressure, velocities and oxygen saturation in the cardiovascular system, pH in the gastrointestinal tract, or electrophysiological properties in cardiac magnetic resonance imaging for diagnosis and treatment of arrhythmia. Furthermore, any kind of image-guided intervention not only requires a fast or often real-time localization of the interventional device, but also sensor measurements might be required, such as contact pressure of an ablation catheter, or the opening angle of a bioptome.

In all these examples, a wireless localization, actuation and communication with the sensor or actuator, is highly desirable, as it would not only facilitate minimally invasive examinations (e.g., swallowing for examinations of the gastrointestinal tract), but also eliminates potential safety hazards related to the presence of electric wires exceeding critical lengths in magnetic resonance imaging (MRI) environment.

Catheter interventions in large magnetic field such as in MRI environment, make use of MRI-safe catheters having a "safe" signal transmission, which comprises multiple short wire segments that are connected via transformers, rendering the catheter shafts rather bulky, difficult to maneuver, and expensive.

A review on device tracking in MRI is presented in S. Kos, et. al., "MR-guided endovascular interventions: a comprehensive review on techniques and applications," Eur. Radiol., vol. 18, no. 4, pp. 645-657, Apr. 2008, doi: 10.1007/s00330-007-0818-4, which is incorporated by reference herein in its entirety.

The information included in this Background section of the specification, including any references cited herein and any description or discussion thereof, is included for technical reference purposes only and is not to be regarded as subject-matter by which the scope of the disclosure is to be bound.

## SUMMARY

The objective of the invention is to provide an improved solution for device localization within an enhanced workflow related to magnetic resonance (MR) diagnostics and treatments.

The invention facilitates wireless interaction of a marker, e.g., localization of the marker, and communication of the marker with the magnetic resonance imaging system, without the need for direct electrical connection through galvanic or optical means, in a standard clinical MRI system.

Furthermore, the invention facilitates a fast or real-time interaction and localization of the marker in an arbitrary imaging sequence during magnetic resonance imaging, hence without need for an additional dedicated sequence or procedure.

In one aspect, the invention provides a method of localizing a marker in a magnetic resonance imaging system, comprising:

generating during a magnetic resonance imaging sequence at least two radiofrequency pulses, wherein at least one is associated to magnetic resonance imaging and at least one that is associated to localization of the marker in a magnetic resonance imaging field, and which is distinct from the at least one radiofrequency pulse associated to magnetic resonance imaging;

generating, by the at least one radiofrequency pulse associated to localization of the marker, a signal receivable during data acquisition of the magnetic resonance imaging sequence;

receiving data from the magnetic resonance imaging field during the duration of data acquisition period within the magnetic resonance imaging sequence;

deriving magnetic resonance imaging data and marker localization data from the received data;

outputting the magnetic resonance imaging data and the marker localization data for the magnetic resonance imaging field.

In some embodiments, the at least one radiofrequency pulse associated to localization of the marker is also associated to communicating with the marker. The communication with the marker besides activating it with the at least one radiofrequency pulse associated to the localization purposes comprised within the magnetic resonance imaging sequence, may be one-way communication or two-way communication. Sending information is beneficial for example to take a measurement at a moment at a current position and not emitting anything until requested a new measurement. If a marker or a sensor to which the marker is applied to, is configured to provide measurements of multiple parameters, it may be specified which one of the measurements is needed. Additionally or alternatively, the marker or an actuator to which the marker is applied to, may be instructed to release a drug, a radiation seed or perform a predefined therapy.

The marker may be passive marker. The marker according to any of the embodiments may be applied to or attached to a medical device, e.g., catheter, guidewire, actuator, sensor device, etc.

In some embodiments the marker is the sensor device, providing physiological parameters transmitted to and received by the same set or a different set of coil elements, i.e. of coils arrays or coil matrix integrated into a flexible mat or rigid structure, used for marker localization, within the duration of data acquisition of the magnetic resonance imaging sequence.

In some embodiments the physiological parameters may be derived directly from the marker localization data, such as for example heartbeat cycle, pulse, breathing frequency.

The marker may further be configured to provide physiological parameter measurement through similar transmission technique of the measurement data like used for the marker localization.

Additionally or alternatively, the sensor device may provide physiological parameters through other wireless transmission principles, for example by using additional readout of measured physiological parameters receivable outside the duration of data acquisition of the magnetic resonance imaging sequence, for example by same coil elements, i.e. of coils arrays or coil matrix integrated into a flexible mat or rigid structure, used for the marker localization that is applied onto the sensor device or by additional coil elements dedicated for readout of signal associated to measured physiological parameters.

Further method embodiments are disclosed and discussed in the detailed description of the invention.

In a further aspect the invention provides a system for localizing a marker in a magnetic resonance imaging system, comprising a computer system configured to:

generate a magnetic resonance imaging sequence during which at least two radiofrequency pulses are emitted, wherein at least one is associated to magnetic resonance imaging and at least one is associated to localization of the marker in a magnetic resonance imaging field, and which is distinct from the at least one radiofrequency pulse associated to magnetic resonance imaging;
receive data from the magnetic resonance imaging field during the duration of data acquisition period within the magnetic resonance imaging sequence, derive magnetic resonance imaging data and marker localization data from the received data, wherein the marker localization data is originating from the marker located in the magnetic resonance imaging field, wherein the marker is configured responsive to the at least one radiofrequency pulse associated to localization of the marker for emitting a signal receivable during data acquisition of the magnetic resonance imaging sequence;
output the magnetic resonance imaging data and the marker localization data for the magnetic resonance imaging field.

In some embodiments, the at least one radiofrequency pulse associated to localization of the marker is also associated to communicating with the marker.

Further system embodiments are disclosed and discussed in the detailed description of the invention.

The system may comprise a memory for storing machine executable instructions, wherein execution of the machine executable instructions causes the computational system to perform any of the method embodiments.

The system may further comprise a magnetic resonance imaging system for providing the magnetic resonance imaging; and a display screen configured to display the information output by the computational system.

In yet a further aspect of the invention a computer program is presented, comprising machine executable instructions, wherein execution of the machine executable instructions causes a computational system to perform any of the method embodiments.

Features described above with respect to method embodiments are applicable and are contemplated also for corresponding system embodiments, with similar benefits.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the system embodiment and method embodiments, as defined in the claims, is provided in the following written description of various embodiments of the disclosure and illustrated in the accompanying drawings.

**BRIEF DESCRIPTION OF THE DRAWINGS**

Illustrative embodiments of the present disclosure will be described with reference to the accompanying drawings, of which:

Figure 1 illustrates an example of a system according to the invention;
Figure 2 schematically illustrates an embodiment of a marker;
Figure 3 schematically and exemplarily shows a circuit diagram of a marker;
Figure 4 illustrates an exemplary magnetic resonance imaging pulse sequence;
Figure 5 illustrates parameter setting for an exemplary experiment according to the invention;
Figure 6 shows an exemplary image acquired with simultaneous magnetic resonance imaging and marker localization upon using dual excitation pulses in the magnetic resonance imaging pulse sequence;
Figure 7 shows magnetic resonance imaging and marker localization signals;

Figure 8 illustrates an exemplary experiment of tracking a marker, according to the invention;
Figure 9 shows results of a further exemplary experiment according to the invention;
Figure 10 illustrates an exemplary method according to the invention.

## DETAILED DESCRIPTION

For the purposes of promoting and understanding the principles of the present disclosure, reference will now be made to the embodiments illustrated in the drawings, and specific language will be used to describe the same. It is nevertheless understood that no limitation to the scope of the disclosure is intended. Any alteration and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately. Features described in relation to a system, may be implemented in a computer implemented method and/or in a computer program product, in a corresponding manner.

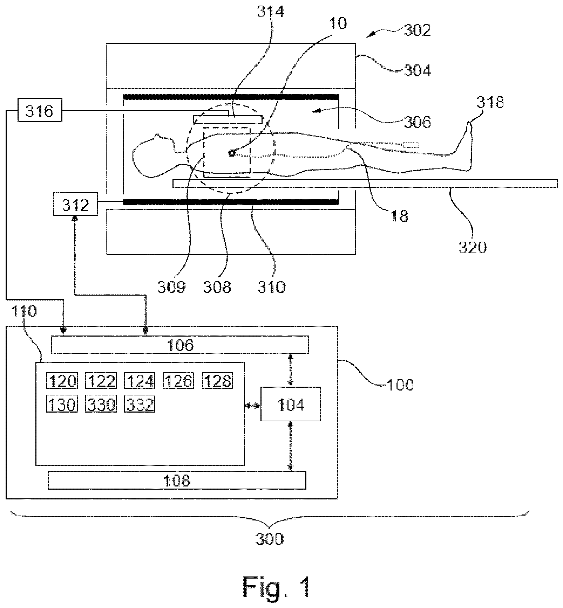

Fig. 1 illustrates an example of a system 300, which may be a medical system. The medical system 300 comprises the computer system 100 and a magnetic resonance imaging (MRI) system 302, or with other words a magnetic resonance imaging scanner. The computer system 100 may be termed control and/or processing system 100, whereas both the computer system and the MRI system may also be termed sub-systems.

The magnetic resonance imaging system 302 comprises a magnet 304. The magnet 304 is a superconducting cylindrical-type magnet with a bore 306 through it. The use of different types of magnets is also possible; for instance, it is also possible to use both a split cylindrical magnet and a so-called open magnet. A split cylindrical magnet is similar to a standard cylindrical magnet, except that the cryostat has been split into two sections to allow access to the iso-plane of the magnet. Such magnets may for instance be used in conjunction with charged particle beam therapy. An open magnet has two magnet sections, one above the other with a space in-between that is large enough to receive a subject: the arrangement of the two sections is similar to that of a Helmholtz coil. Open magnets are popular because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A region of interest 309 is shown within the imaging zone 308. Magnetic resonance data is typically acquired for the region of interest. A subject 318 is shown as being supported by a subject support 320 such that at least a portion of the subject 318 is within the imaging zone 308 and the region of interest 309.

Within the bore 306 of the magnet there is also a set of magnetic field gradient coils 310, which is used for acquisition of (preliminary) magnetic resonance data to spatially encode magnetic spins within the imaging zone 308 of the magnet 304. The magnetic field gradient coils 310 are connected to a magnetic field gradient coil power supply 312. The magnetic field gradient coils 310 are intended to be representative. Typically, magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308, there is a radiofrequency coil 314 for manipulating the orientations of magnetic spins within the imaging zone 308 and also for receiving radio transmissions from spins within the imaging zone 308. The radiofrequency coil may also be referred to as a channel or antenna. The radiofrequency antenna may contain multiple coil elements. The coil elements may be integrated in a flexible mat, in a rigid structure or their hybrid combination. Multiple coil elements support coil sensitivity encoding of position for localization of a marker 10 or multiple markers. The radiofrequency coil 314 is connected to a radiofrequency transceiver 316. The radiofrequency coil 314 and radio frequency transceiver 316 may be replaced by separate transmit and receive coils and a separate transmitter and receiver. It is understood that the radiofrequency coil 314 and the radiofrequency transceiver 316 are representative. The radiofrequency coil 314 is intended to also represent a dedicated transmit antenna and a dedicated receive antenna. Likewise, the transceiver 316 may also represent a separate transmitter and receivers. The radiofrequency coil 314 may also have multiple receive/transmit elements and the radio frequency transceiver 316 may have multiple receive/transmit channels.

The transceiver 316 and the gradient controller 312 are shown as being connected to the hardware interface 106 of the computer system 100.

In this example, the computer system 100, which can further be implemented as a control and processing console of the MRI system, may represent one or more computer systems that are in one location or are distributed. The computer system 100 is shown as including a computational system 104. The computational system 104 may for example be one or more processing cores that are located at one or more locations. The computational system 104 is shown as being connected to an optional hardware interface 106. The hardware interface 106 may for example be used to connect the computational system 104 with other components of the computer system 100 if they are present. The hardware interface 106 may enable the computational system 104 to control such components.

The computer system is further shown as comprising an optional user interface 108. The user interface 108 may for example be used by an operator to control the operation and function of the computer system 100. The computer system 100 could be a standalone computer system but it could also be integrated into a magnetic resonance imaging system.

The computer system is further shown as comprising a memory 110. The memory 110 is intended to represent any combination of memory or storage device which is accessible to the computational system 104. The memory 110 may include volatile and non-volatile memory storage means and components. The memory 110 in some embodiments may be based on or may rely on cloud-based data stored in logical pools across disparate, commodity storage servers located on premises or in a data center managed by a third-party cloud provider. Accordingly, the memory as schematically depicted in Fig. 1 is just for illustration purposes. Therefore, any or all components comprised in memory 110 may be cloud-based.

The memory 110 is shown as containing machine-executable instructions 120. The machine-executable instructions 120 may be configured to enable the computational system 104 to perform basic data processing and image processing tasks, such as reconstructing magnetic resonance images. The machine-executable instructions 120 may also in some examples contain code, which enables the computational system to control other components via the optional hardware interface 106.

The memory 110 may comprise image segmentation algorithm 122. The image segmentation algorithm 122 may be a standard magnetic resonance imaging image segmentation algorithm that is configured for outputting one or more predetermined anatomical regions for magnetic resonance imaging data. This may be for a particular field of view or anatomical region of the subject, e.g., one or multiple anatomical structures or organs of a patient.

The memory 110 may comprise or store the measured magnetic resonance imaging data 124. The memory 110 may further comprise an image segmentation 126 that has been received from the image segmentation algorithm 122 by inputting the measured magnetic resonance imaging data 124. The memory 110 may comprise a selected image portion 128. This is one or more regions or anatomical regions that have been identified in the image segmentation 126. The selected image portion 128 may for example be selected using a predetermined criterion that is applied to the image segmentation 126. The memory may comprise one or a plurality of various algorithms 130 for deriving position and/or orientation of a medical device or marker 10 within the region of interest 309 of an imaging zone 308 based on magnetic resonance imaging data 124 and by optionally using any information present in the memory 110 and/or information that may be input by a user through the user interface 108.

The memory 110 is shown as containing pulse sequence commands 330. The pulse sequence commands are commands or data, which can be converted into commands that can be used to control the magnetic resonance imaging system 302 to acquire k-space data 332. Examples of pulse sequence comprised by the pulse sequence commands 330 may be any of a spin-echo imaging sequence, a gradient-echo imaging sequence and one of their hybrid combination.

The memory 110 is further shown as comprising k-space data 332 that has been acquired by controlling the magnetic resonance imaging system 302 with the pulse sequence commands 330. The computational system 104 may also reconstruct the measured magnetic resonance imaging data 124 from the k-space data 332.

The marker 10 may be configured to be attached to, integrated into or applied to a device, in particular a medical device, which may for example be an interventional device 18 for diagnostic or therapy purpose, such as a catheter, a guidewire, an implantable device like stent, left atrial appendage closure device, blood clot filter, etc., of which medical devices it is desirable or necessary to know the position and/or orientation relative to anatomical structures such as organs (e.g., heart, brain, etc.) and/or ducts (e.g., blood vessels, pancreatic duct, etc.). In the context of the invention ascertaining the position of the marker or the sensor device is understood as localization, whereas ascertaining the position of the marker or sensor device for successive moments in time is understood as tracking.

The wireless marker 10 requires a miniaturized receive antenna or coil element, which allows to energize the device via external electromagnetic fields, such as a radiofrequency (RF) excitation pulse employed in MR imaging. The resonance frequency of this receiver should be within the bandwidth of the imaging system, but with an offset with respect to the bandwidth of the current imaging sequence, in order to allow for a spectral separation of the magnetic resonance imaging signal and sensor signal. In an example embodiment the offset is 1 kHz for a 1.5T MRI system with a Larmor frequency of about 64 MHz. The offset may be larger, for example below +30 kHz, such as +5 kHz, +1 kHz, or may be negative, with similar values. The offset may be provided by specification of a predetermined percentage with respect to the Larmor frequency of the magnetic resonance imaging system, either with a negative or positive sign, e.g., below 0.05 %, 0.01 %, 0.005 %, 0.002 %, etc.

Furthermore, the sensor device to which the marker is applied to may comprise an electronic circuit that conducts the physiological measurement (e.g., pressure, pH, blood flow etc.), and configured to emit a response signal within the same frequency band, via the same coil element or antenna.

Additionally or alternatively, the sensor device or marker may provide at least one physiological parameter comprising parameters related to heartbeat and/or breathing.

The actual measurement information from the sensor device, or reverse communication/instruction towards the sensor device is implemented via an amplitude-, phase-or frequency-modulation of the emitted RF pulses.

The marker may be a sensing unit comprising a resonator element having piezoelectric properties and a coil element, wherein the coil element may be configured to transduce an external magnetic or electromagnetic excitation field into an output voltage to be provided to the resonator element and the resonator element may be configured to transduce the output voltage into respective mechanical oscillations in a resonant mode and to provide a piezoelectric voltage to the coil element. The coil element may then be configured to transduce the piezoelectric voltage into a magnetic field to be detected by a position tracking array, such as the radiofrequency coil, in the position tracking system, e.g., MRI system.

The magnetic coil element, with other words the coil resonator, may be combined with an energy-storing oscillator, in terms of the resonator element having piezoelectric properties. The externally applied magnetic or electromagnetic excitation field causes mechanical oscillations at the resonance frequency of the resonator element. The oscillation is persistent due to the high-quality factor of the circuit. This quality factor may particularly be a quality factor having a value above $10^3$ or even higher.

The coil element and the resonator element may particularly be electrically connected to one another. The coil element may particularly comprise or be made of copper. The coil element may, alternatively or additionally, comprise or be made of silver. In some embodiments, in particular embodiments where the marker device is supposed to be transparent to radiation, the coil element may comprise or be made of aluminum. In some embodiments, in particular embodiments where the marker device is supposed to be opaque to radiation, gold may be chosen for the windings to serve dual purpose.

Further, the term resonator element may, in this context, be understood as corresponding to an element that is connected to the coil element in order to respond to the coil element's voltage output in response to an externally applied magnetic or electromagnetic field by absorbing energy, starting to perform an electrical or mechanical oscillation. The resonator element may for example comprise a crystal, such as a quartz crystal, though it may comprise additionally or alternatively a different material, such as certain ceramics known in the art with appropriate properties required by the technical implementation and/or such as piezoelectric polymer, e.g., polyvinylidene fluoride (PVDF) or polyvinylidene fluoride trifluoroethylene (P(VDF-TrFE)).

The oscillations of the resonator device may particularly be provided in resonant mode. This is to be understood as meaning that the resonator device may, in response to the output voltage applied to it, start oscillating at or near to its resonance frequency. In some embodiments, this is achieved by having the externally applied magnetic or electromagnetic field provided with the right frequency components to lead to an output voltage of the coil element that achieves such resonant oscillations. Due to the resonator element oscillating at its resonance frequency, it acts as an energy storage even in cases where no output voltage is applied anymore. This reduces the damping of the resonant circuit formed by the coil element and the resonator element.

The resonator element may be electrically connected to the coil element via respective contacts.

Further, the resonator element may have piezoelectric properties. In this context, the term piezoelectric properties shall be understood within the conventional meaning, for example, as describing a material which deforms in response to an electrical voltage being applied thereto and which, in response to the mechanical stress exerted by the deformation accumulates electric charge that may be output in terms of a piezoelectric voltage. The accumulation of electric charge may hereby happen in a reversible manner, changes in mechanical stress from a first to a second state may lead to the electric charge being accumulated and changes from the second to the first state in mechanical stress will result in the material having the same (electric) properties as before again.

The term coil element may particularly refer to an element comprising and/or corresponding to a radiofrequency signal receive coil arrangement. The coil element may be an off-the-shelf coil having an appropriate receive bandwidth and Q-factor. The size and geometry of the coil may hereby particularly, be determined based on the desired receive properties. In some embodiments, particularly in embodiments with space constraints, the resonator element may be provided inside the coil element and may be electrically connected to the contacts of the coil element. Alternatively, the resonator element may be provided at some distance away from the coil element while still being electrically connected thereto via respective contacts. The coil element may particularly be provided by winding a coil around the resonator element, whereby the coil is wound in a manner such that it does not touch the resonator element.

The coil element may be arranged at a distance from the resonator element. The resonator element and the coil element may, for this purpose, be connected via a respective connection portion. In some embodiments, particularly in embodiments with space constraints, the distance may be achieved by providing the windings around the resonator element such that there is a space between the windings of the coil element and the resonator element. The dimensions of this space should hereby be chosen appropriately according to the dimensioning of the marker device. Hereby, it should be noted that the windings of the coil element become more efficient when they are further away from a rotational axis of the coil element. The arrangement between the resonator element and the coil element may therefore be such that the resonator element may be provided in the coil element and extends along its axis.

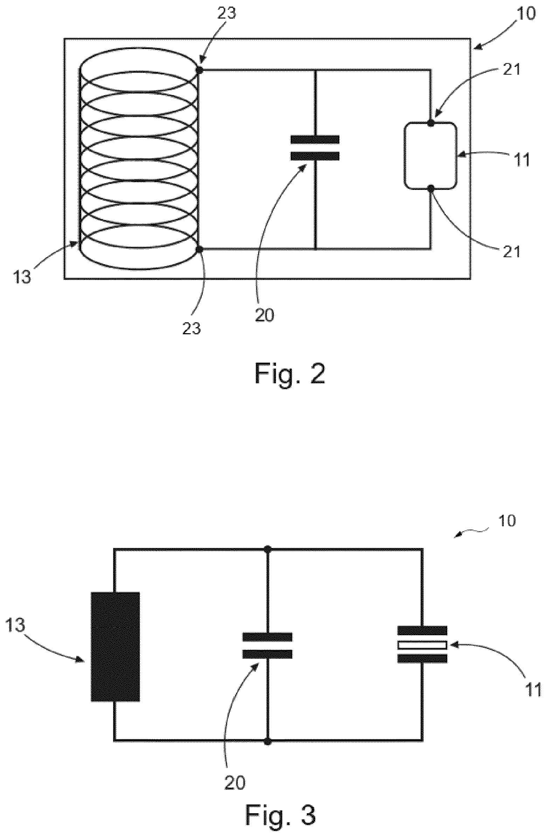

Fig. 2 schematically and exemplarily illustrates an embodiment of a marker 10, which may be a sensor device itself. The marker is configured to be attached to, applied to or integrated into an interventional device 18 or an implantable device as presented in general hereinbefore. The marker 10 comprises, in the specific embodiment according to Fig. 2, a resonator element 11, a coil element 13 and a capacitive element 20. The resonator element 11 and the coil element 13 are hereby connected with each other in series. Further, the capacitive element 20 is connected in parallel to the coil element.

The capacitive element is not necessary in all embodiments, however the function of the capacitor is needed. The capacitance may just consist of the stray/parasitic capacitances of the coil, resonator and connecting leads.

The radiofrequency coil 314 is configured to provide an electromagnetic excitation field having particular frequency components. This electromagnetic excitation field acts on the coil element 13.

The coil element 13 transduces the electromagnetic excitation field into a respective output voltage having a particular frequency, which is close to the resonance frequency of the resonator element 11.

The output voltage is then provided, via terminals 23 of the coil element to terminals 21 of the resonator element 11 via a respective electrical connection. Hereby, the output voltage, prior to being fed to the resonator element 11, may be amplified by the capacitive element 20. That is, capacitive element 20 is provided to have a capacitance that allows amplification of the output voltage provided by the coil element 13 to resonator element 11. The output voltage is then received by the resonator element 11 and effects a deformation therein.

The resonator element 11 may comprise any of the material presented hereinbefore. In response to receiving the adjusted output voltage from the coil element 13, the resonator element 11 is deformed. Due to the frequency component being within a resonant frequency of the resonator element 11, the deformation is excited in the resonant mode. As such, the resonator element 11 starts performing mechanical oscillations in a resonant mode, leading to the oscillations being persistent for a while, even if no output voltage is provided from the coil.

Deformation of the resonator element 11 hereby leads to a voltage being generated by the resonator element 11 due to its piezoelectric properties. The generated voltage, with other words the piezoelectric voltage in the following, is then provided to the coil element 13, resulting in a current being generated through the coil element 13. This current causes generation of an electromagnetic field in the coil element 13. This resulting electromagnetic field may then be detected by the radiofrequency coil 314 and used for position determination for marker localization or tracking. Due to the oscillations being persistent, even without the resonator element 11 receiving the output voltage, the resonator element 11 acts as an energy storage, continuing providing the piezoelectric voltage to the coil element 13. This leads to the current being continuously generated through the coil element 13, meaning that the coil element 13 continues providing an electromagnetic field that may be detected by the radiofrequency coil 314. Accordingly, by using the resonator element 11 as an energy storage, the damping of the resonant circuit formed by the resonator element 11, the capacitive element 20 and the coil element 13 may be educed, meaning that the circuit has a high quality factor.

Fig. 3 schematically and exemplarily shows a circuit diagram representative of the resonant circuit formed by the resonator element 11, the coil element 13 and the capacitive element 20. As shown in the circuit diagram, the resonator element 11 and the coil element 13 are connected in series and the capacitive element 20 is connected in parallel to the coil element 13.

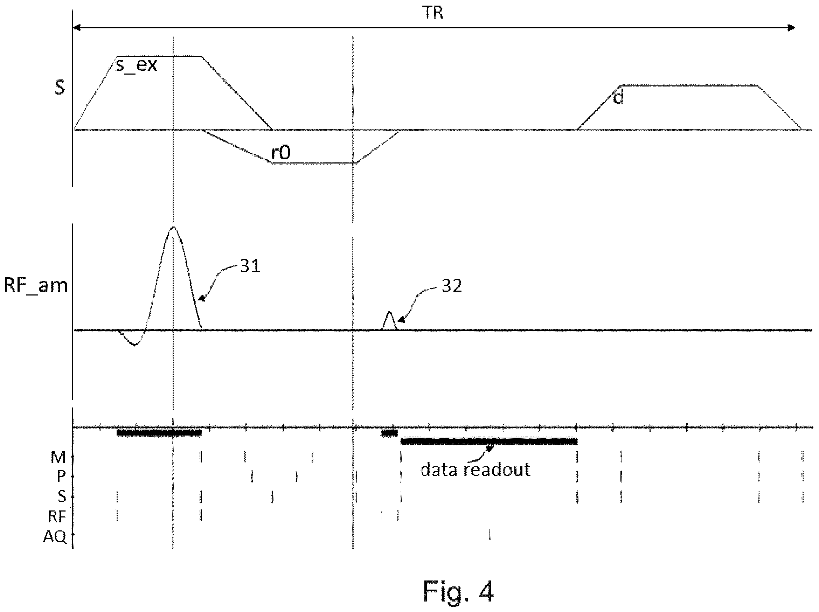

According to the invention, a dedicated RF excitation pulse, a marker excitation pulse 32, that matches the resonant frequency of the marker is included during the MRI sequence, in addition to the standard excitation pulse 31, see for example in Fig. 4. In the example that is provided in Fig. 4 with a nonlimiting scope, s_ex is slice selection gradient during RF excitation; r0 is corresponding rephasing gradient; d is dephasing gradient at end of sequence; RF is radiofrequency excitation pulse; AQ is data acquisition window; M is measurement; P is phase-encode; S is slice-select spatial directions. Apart from the spectral separation (frequency offset), the amplitude of the additional pulse 32 is typically very small, typically below $1\mu T$, as the absorption/transmission efficiency of the marker is some orders of magnitude higher than that of the actual proton resonance. In MRI terms, the "flip angle" of the additional RF pulse is in the order of millidegrees. Therefore, it can be included during any clinical MRI sequence without interference with the actual MR imaging. Typically, the additional RF pulse is timed just before the start of the MRI data sampling or data acquisition thus that the energizing or transmission processes to the marker is completed, and the response signal is emitted during MRI data sampling. The time is only limited by the hardware of the MRI system. Ideally it should be less than 1 ms, preferably less than 100 $\mu s$, more preferably even 10 $\mu s$. Typical values for clinical systems are around 100 $\mu s$. This marker excitation pulse may be played or included in every repetition of the MR imaging sequence, though it is not necessary to use in all repetitions of the MRI sequence the marker excitation pulse 32, in case localization of the marker is sufficient at predetermined time intervals that exceeds the duration of a single MR imaging sequence. The predetermined time intervals may be regular or irregular time intervals. Nevertheless, it may have particular benefit to include the marker excitation pulse 32 in each consecutive MR imaging sequence, yielding a marker localization or marker position reading every few milliseconds.

The receive data comprises the magnetic resonance information and marker signal, preferably recorded by using a multi-element coil array or coil matrix, which magnetic resonance information and marker signal are separated in the spectral domain. The magnetic resonance information or with other words magnetic resonance imaging data, is fed into the regular image reconstruction process, whereas the marker signal which comprises the marker position information, undergoes a dedicated algorithm, which demodulates the sensor information (measurement / reading), and performs the localization of the marker based on the data received from the radiofrequency coil array. The marker localization algorithm is equivalent to solving an inverse problem, for example, what is the most likely marker position and orientation that would yield the signal received in this coil array. This algorithm can be implemented efficiently using e.g., tensor operations on a graphics processing unit (GPU), or by using a pre-trained neuronal network. In fact, the marker localization is based on the sensitivity of the coils. If the marker is nearer and better aligned to one specific receive coil, the signal is stronger. Each coil has a sensitivity profile, which is a vector value field. The marker also has a vector quantity, which is the dynamic dipole moment. Now with all possible positions in space and orientation, the scalar product of sensitivity and dynamic dipole moment is computed. The position with the best fit is returned as the most likely position. Best fit may include the fitting of the amplitude and phase of the dynamic dipole moment as in general this is also not known. For example, if the complex valued marker signal which is detected by each MR receive coil indexed by $i$ is given by $a_i$ and $c_i(x)$ is the complex valued spatially dependent receive sensitivity of the coils, then x, the position and orientation of the marker, can be determined by solving the following minimization problem:

$$min_{x,\phi,\mu_0}\left(\sum_{i=1}^{n}\left|a_i - c_i(x)\mu_0 e^{i\phi}\right|\right)$$

To solve this problem, a model for the spatial variation of the coil sensitivities is required. There are several ways to obtain such a model:

(i) by using electro-magnetic field calculations based on the geometry (and other physical properties) of the receive coils. This has the advantage that it leads to a patient independent model which needs to be computed only once and can be evaluated at arbitrary positions;

(ii) by using the measured receive sensitivity profiles, which typically are acquired during a normal MR imaging procedure because they are needed for the image reconstruction. This has the advantage that wave propagation effects induced by the patient (which can be significant especially at high field strengths) are accurately represented. A disadvantage is that the model may only be evaluated at locations with MR signal, which limits the region where marker tracking is possible. This means in particular, marker tracking is not possible outside the patient's body and in some anatomical regions which give no substantial MR signal (e.g., inside the lung or air-filled cavities like esophagus or sinuses).

(iii) by using a fusion of the previous two methods, which uses electromagnetic modeling based on coil geometry and electromagnetic properties of the patient (e.g., derived from MR measurements). This removes the disadvantages of both previous methods but is much more complex than the other methods and as a result, somewhat slower in implementation.

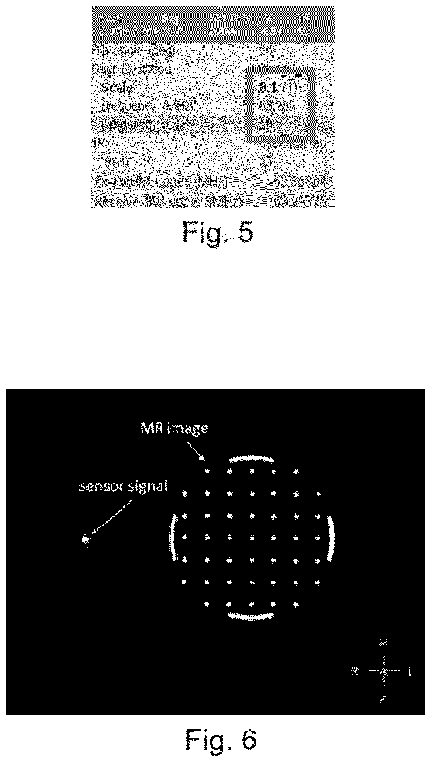

A proof of principle experiment has been performed, wherein a sensor device or marker according to Fig. 2 or Fig. 3 has been used with a dual pulse 31 and 32 conform Fig. 4 in a 1.5T MRI system. The additional marker excitation pulse 32 with small flip angle, which in the nonlimiting proof of principle example has been chosen 0.1 degree, has been specified via the frequency, bandwidth and scale, as illustrated in Fig. 5. Additionally, measures have been taken to ensure that the two pulse frequencies are spectrally separated, but within the received bandwidth of the MRI system. An exemplary image acquired with simultaneous MR imaging and marker localization upon using dual excitation pulse is shown for illustration in Fig. 6, wherein cross-section MR image of a resolution phantom was acquired with simultaneous excitation on the marker resonance frequency. The frequencies and bandwidths were selected such that the marker detection signal appears at the edge of the visible field of view (FOV). No interference between the MR imaging and marker localization signal has been observed. The marker excitation pulse is played with every repetition time (TR), yielding marker signal readouts at a very high rate, with intervals in the order of a few milliseconds, which may be termed real-time in medical device localization and tracking, because this time-interval of refreshing current localization information is orders of magnitude lower than the fastest recorded human reaction times to visual stimuli, e.g., reaction to a visual illustration based on which a user takes an action either to reposition the marker or a sensor device, to measure a physiological or anatomical parameter or to apply therapy associated to the location of the sensor, is around 120 milliseconds, which is sufficient for a time-resolved measurement of most biological or physiological processes.

Alternatively, in case localization of the marker or a device on which the marker is integrated is not necessary every few milliseconds, the marker excitation pulse can be used from time to time in the repeated MR imaging sequences, such as for example at every 5th, 10th, 100th, etc.

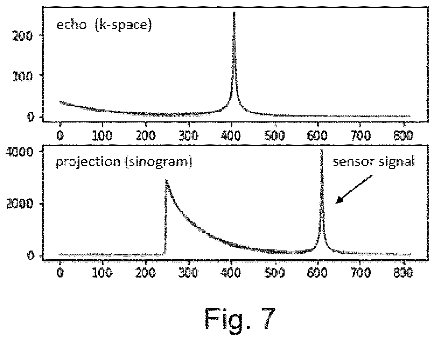

The MR imaging and sensor device localization signals for one selected readout associated to one TR are illustrated in Fig. 7. The upper graph represents the receive data (k-space echo), and the lower graph represents the Fourier-transformed sinogram of one readout, therewith the MR imaging data and sensor localization signals are clearly separated.

After transformation into the spatial domain (Fourier transform), the sensor localization signal is distinguishable as a distinct side-peak of the MR sinogram, making a spectral separation of MR imaging data and sensor localization data easily separable. To implement communication between the MR system and the marker, the RF pulses transmitted from the MR system to the marker, and the response signal may be modulated in amplitude, phase, and/or frequency.

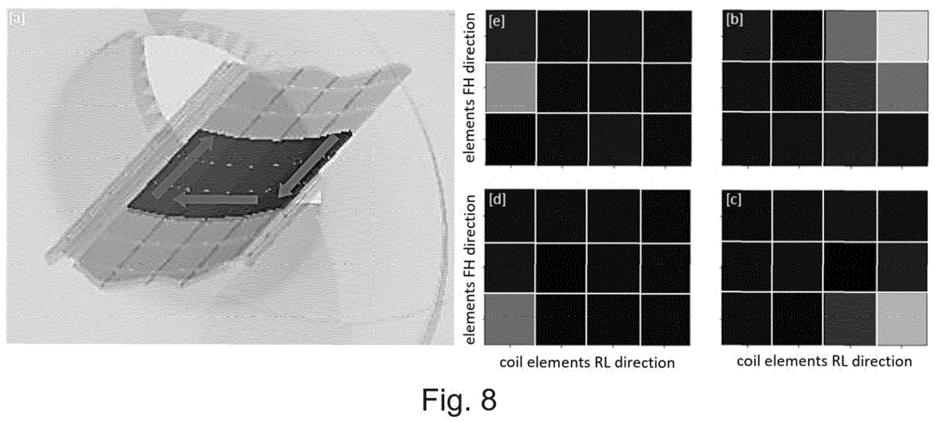

For localization of a device or tracking the motion in space and time within the bore of an MRI system, wherein the device comprises the marker as discussed in any of the embodiments according to the invention, simultaneous signal reception with multiple coil elements or with other words a coil array or matrix may be used. In the following example, the standard posterior coil array of the MRI system is used. Fig. 8 illustrates in image [a] a scanner-integrated posterior receive coil matrix with 4x3 elements used for simultaneous reception of MR imaging data and marker localization signals. For illustration, the marker was moved in U-shape trajectory, indicated with arrows, over the coil array during the acquisition of an MR image. In images [b]-[e] the (separated) sensor signal amplitudes are shown, which were received via the respective coil elements within the 4x3 array at different times during the movement according to the arrows in image [a]. First, the marker has been moved from head to feet direction (HF), then from right to left (RL), then from feet to head direction (FH).

For an accurate localization of the marker position the complex marker localization signals, e.g., amplitude and phase, received with the individual coil elements from the coil matrix require a processing step similar to solving an inverse problem with respect to: which is the most likely position of and orientation of the marker or sensor that will induce the specific signal that is received in the coil array, that has already been discussed hereinbefore.

For a coarse estimation of sensitivity to motion (smallest detectable motion amplitude), an elongate device with the attached marker was placed on a soft pad, and the elongate device was periodically tipped with the finger in order mimic small motion amplitudes (<1 mm), similar to microscopic chest-wall motion induced by bulk cardiac (contractile) motion.

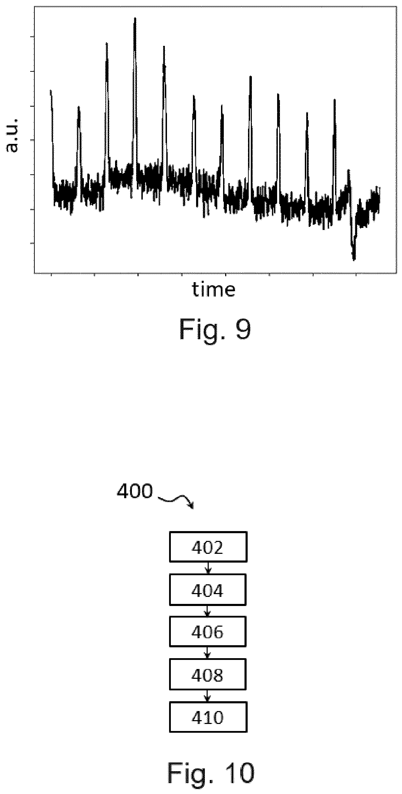

The evolution of the sensor localization signal phase for the coil with the strongest receive signal over image acquisition duration, approximately 12 seconds is plotted in Fig. 9, wherein the periodic blips are distinguishable in the sensor signal phase, which are related to microscopic sensor motion. The x-axis represents time and y-axis represents phase in arbitrary units, which in this case is a measure for amplitude. Read-out is simply the recorded (complex) amplitude of a specific coil at a specific frequency during the data acquisition phase.

This data suggests that, in an additional or alternative embodiment, the present invention can be used as a wireless detector for cardiac motion and/or respiratory motion. The data shows that there is a measurable signal associated with a movement of the marker with an amplitude, likely much smaller than 100 μm. The most straightforward method to translate this possibility into the detection of heartbeat and breathing motion is to fit the output data, here the position data, to a suitable model of motion due to heartbeat or breathing. This would directly identify motion by the timing and amplitude of signal peaks as observed in Fig. 9, so that the signal may also be used for controlling the dynamics of the MRI scan using respiratory triggering or cardiac gating. Another approach is to perform a Fourier transformation of a longer signal trace, in which the heart rate and respiratory rate can be identified as distinct spectral signal peaks. Additionally or alternatively, peak detection algorithms can be used, such as disclosed in WO2017/129495 A1, which is incorporated by reference herein in its entirety.

In a further aspect of the invention with reference to Fig. 10, a method 400 of localizing a marker in a magnetic resonance imaging system is disclosed. Upon generating 402, during a magnetic resonance imaging sequence at least two radiofrequency pulses 31 and 32, the marker is excited by the at least one pulse 32 that is associated to localization of the marker in a magnetic resonance imaging field. The at least one other pulse 31 is associated to magnetic resonance imaging, and it is distinct from the at least one radiofrequency pulse associated to localization of the marker. The magnetic resonance imaging sequence may be any sequence used in magnetic resonance imaging, including any spin-echo-based imaging sequence, any gradient-echo-based imaging sequence and any of their hybrid combination. The at least one radiofrequency pulse 32 associated to localization of the marker generates 404 a signal in the marker, which is receivable during data acquisition of the magnetic resonance imaging sequence, already discussed with reference to Figs. 2 to 4. Generating the signal in the marker is realized by activation of the marker by the at least one pulse 32 that is associated to localization of the marker. Therefore, the at least one radiofrequency pulse associated to localization of the marker is also associated to communicating with the marker, because the marker is activated by the at least one radiofrequency pulse 32, thereby the MRI system is communicating with the marker through the radiofrequency coil 314, which then receives during data acquisition of the magnetic resonance imaging sequence also the signal that has been generated in the marker upon the at least one radiofrequency pulse 32. Hence, communication between the radiofrequency coil 314 and the marker 10 is two-way communication, comprising transmission for activation of the marker and reception for signal generated by the marker from which either the location of the marker is derived, or the location of the marker and physiological parameters are derived.

The data is received 406 from the magnetic resonance imaging field during the duration of data acquisition period, i.e. data sampling period, within the magnetic resonance imaging sequence. The magnetic resonance imaging data and marker localization data are derived 408 from the received data, which subsequently are output 410 for example to peripheral devices or further computing systems.

In some embodiments of the method outputting comprises a graphical representation of a magnetic resonance image based on the magnetic resonance imaging data and the marker localization data.

In some embodiments the method may further comprise: deriving at least one physiological parameter from the received data besides the magnetic resonance imaging data and marker localization data; outputting additionally the at least one physiological parameter. The at least one physiological parameter may comprise heart-beat cycle, frequency of breathing.

In some embodiments the marker may provide physiological parameters through similar transmission technique of the measurement data like used for the marker localization. In one of the embodiments the information is encoded in the resonance frequency of a crystal resonator. The simplest is to use the temperature drift of the resonance frequency as a measure for the temperature at the marker. A more general approach is to use the resonator as a sensor similar to quartz crystal microbalances, e.g., https://en.wikipedia.org/wiki/Quartz_crystal_microbalance, as a starting point for this rather large field in metrology. For example, moisture can be measured when coating the crystal with a moderately hygroscopic polymer like nylon. The crystal enables all sorts of concentration measurements. Also, with a suitable adsorber layer pressure can be measured by a pressure mediated adsorption of a suitable gas, usually, a gas with a boiling point quite close to the operation temperature. In general, the adsorber layer needs to interact with the quantity to be measured by changing its mass, e.g., by absorption of humidity, or simply compression by variable partial gas pressures. As one example, a suitable adsorber layer would be polyamid (for moisture sensing), or latex for pressure sensing if it enclosed by a suitable gas, e.g., butan in a compressible vessel / container.

In some of the embodiments of the method deriving marker localization data is according to spatial and temporal domains; and outputting the magnetic resonance imaging data comprises successive temporal sequence of marker localization data for the magnetic resonance imaging field.

In some embodiments of the method the signal generated by the marker upon reception of the at least one radiofrequency pulse associated to localization of the marker, is within the bandwidth of the magnetic resonance imaging system, and is offset with respect to the bandwidth of the magnetic resonance imaging sequence. In some embodiments the offset is below 0.05 % of the Larmor frequency of the magnetic resonance imaging system. In some embodiments the offset is below 0.002 % of the Larmor frequency of the magnetic resonance imaging system.

Some embodiments of the method may further comprise: providing an image segmentation comprising one or more anatomical regions within the magnetic resonance imaging data in response to inputting the magnetic resonance imaging data into an image segmentation algorithm. The image segmentation may be provided for the graphical representation of the magnetic resonance image based on the magnetic resonance imaging data and the marker localization data.

In some of the embodiments the marker localization may be related to localization of a portion of a medical device on which the marker is applied.

In some of the embodiments the marker localization data may comprise localization data from a plurality of markers applied on the medical device, and it may further comprise: deriving orientation of the medical device in the magnetic resonance imaging field based on marker localization data associated to at least two of the plurality of markers; and outputting the medical device orientation. If multiple markers are used, they should have in general different frequencies, which could be both lower and higher than the proton frequency used for MRI. The slices of MR imaging do not contribute to the marker separation as the marker localization is totally disjunct from MR imaging. If sufficient number of coils are used, it is possible to separate makers at the same frequency. In general, the position and orientation of one marker is possible with six coils. Accordingly, twelve coils allow separation of position and orientation of two markers at the same frequency. There is also the possibility to separate the markers due to their Q-value (dampening, e.g., measured as spectral linewidth). Although this technique is less efficient than separation in frequency, it allows determination of position and orientation for at least ten markers if the Q values are sufficiently different from each other. In general, already from the position of multiple markers on an interventional instrument like a catheter or guidewire, the shape of the interventional instrument can be inferred, which can be termed as shape sensing. Spatial distribution of multiple markers on an implantable device can also provide valuable information during a clinical procedure, for example when positioning the implantable device with respect to anatomical landmarks.

In a yet further aspect, a computer program for controlling a marker or medical device localization system or tracking system as described above to localize and/or track a marker or medical device as likewise described above is provided, which, when executed by a processing device, is adapted to perform any of the method embodiments as described above. In a further aspect, a computer-readable medium having stored thereon the computer program is provided. The computer-readable medium may for example be a non-transitory computer readable medium.

It shall be understood that the marker localization or tracking system, the method, the computer program, and the computer-readable medium, may have similar and/or corresponding features.

It is understood that one or more of the aforementioned embodiments of the invention may be combined as long as the combined embodiments are not mutually exclusive.

Although various embodiments of the claimed subject matter have been described above with a certain degree of particularity, or with reference to one or more individual embodiments, those skilled in the art could make numerous alterations to the disclosed embodiments without departing from the spirit or scope of the claimed subject matter.

Still other embodiments are contemplated. It is intended that all matter contained in the above description and shown in the accompanying drawings shall be interpreted as illustrative only of particular embodiments and not limiting. Changes in detail or structure may be made without departing from the basic elements of the subject matter as defined in the following claims.

**Claims**

1. A method (400) of localizing a marker in a magnetic resonance imaging system, comprising:

   generating (402) during a magnetic resonance imaging sequence at least two radiofrequency pulses, wherein at least one is associated to magnetic resonance imaging and at least one that is associated to localization of the marker in a magnetic resonance imaging field, and which is distinct from the at least one radiofrequency pulse associated to magnetic resonance imaging;
   generating (404), by the at least one radiofrequency pulse associated to localization of the marker, a signal receivable during data acquisition of the magnetic resonance imaging sequence;
   receiving (406) data from the magnetic resonance imaging field during the duration of data acquisition period within the magnetic resonance imaging sequence;
   deriving (408) magnetic resonance imaging data and marker localization data from the received data;
   outputting (410) the magnetic resonance imaging data and the marker localization data for the magnetic resonance imaging field.

2. The method according to claim 1, wherein the at least one radiofrequency pulse associated to localization of the marker is further associated to communicating with the marker.

3. The method of claim 1 or 2, wherein outputting comprises a graphical representation of a magnetic resonance image based on the magnetic resonance imaging data and the marker localization data.

4. The method of any of the preceding claims, comprising:

   deriving at least one physiological parameter from the received data besides the magnetic resonance imaging data and marker localization data;
   outputting additionally the at least one physiological parameter.

5. The method of any of the preceding claims, wherein the at least one physiological parameter comprises parameters related to heartbeat and/or breathing.

6. The method of any of the preceding claims,

   wherein deriving marker localization data is according to spatial and temporal domains;
   wherein outputting the magnetic resonance imaging data comprises successive temporal sequence of marker localization data for the magnetic resonance imaging field.

7. The method of any of the preceding claims, wherein the signal generated by the marker upon reception of the at least one radiofrequency pulse associated to localization of the marker, is within the bandwidth of the magnetic resonance imaging system, and is offset with respect to the bandwidth of the magnetic resonance imaging sequence.

8. The method of claim 7, wherein the offset is below 0.05 % of the Larmor frequency of the magnetic resonance imaging system, optionally the offset being below 0.002 % of the Larmor frequency of the magnetic resonance imaging system.

9. The method of any of the preceding claims, further comprising:
   providing an image segmentation including one or more anatomical regions within the magnetic resonance imaging data in response to inputting the magnetic resonance imaging data into an image segmentation algorithm.

10. The method of claim 9 when depending on claim 3, wherein image segmentation is provided for the graphical representation of the magnetic resonance image based on the magnetic resonance imaging data and the marker localization data.

11. The method of any of the preceding claims, wherein the marker localization is related to localization of a portion of a medical device on which the marker is applied.

12. The method of claim 11, wherein the marker localization data comprises localization data from a plurality of markers applied on the medical device, wherein the method further comprises:

   deriving orientation of the medical device in the magnetic resonance imaging field based on marker localization data associated to at least two of the plurality of markers; outputting the medical device orientation.

13. A system for localization of a marker in a magnetic resonance imaging system, comprising: a computational system configured to perform a method according to any of the claims 1 to 12.

14. The system of claim 13, further comprising:

   a magnetic resonance imaging system for providing the magnetic resonance imaging data; a display screen configured to display the information output by the computational system.

15. A computer program comprising machine executable instructions, wherein execution of the machine executable instructions causes a computational system to perform a method according to any of the claims 1 to 12.

Fig. 1

Fig. 2

Fig. 3

Fig. 4

Fig. 5

Fig. 6

Fig. 7

Fig. 8

Fig. 9

Fig. 10

EUROPEAN SEARCH REPORT

**Application Number**

EP 24 16 8931

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 745 303 B1 (KONINKLIJKE PHILIPS NV [NL]) 27 September 2017 (2017-09-27) <br> * paragraph [0006] - paragraph [0007] * <br> * paragraph [0012] - paragraph [0018] * <br> * claim 4 * <br> * figures 1,3 * | 1-15 | INV. <br> A61B5/055 <br> A61B5/06 <br> G01R33/28 <br> G01R33/26 <br> G01R33/567 <br> G01R33/36 <br> A61B5/00 |
| A | EP 3 140 667 B1 (KONINKLIJKE PHILIPS NV [NL]) 23 December 2020 (2020-12-23) <br> * paragraph [0008] * | 1-15 | |
| A | EP 2 713 868 B1 (KONINKL PHILIPS NV [NL]) 13 July 2016 (2016-07-13) <br> * paragraph [0036] * | 1-15 | |
| A | US 2010/168553 A1 (MARTEL SYLVAIN [CA] ET AL) 1 July 2010 (2010-07-01) <br> * paragraph [0008] * | 1-15 | |
| A | JOAO JORGE: "Tracking discrete off-resonance markers with three spokes (trackDOTS) for compensation of head motion and B 0 perturbations: Accuracy and performance in anatomical imaging", MAGNETIC RESONANCE IN MEDICINE, [Online] vol. 79, no. 1, 5 March 2017 (2017-03-05), pages 160-171, XP093159038, US ISSN: 0740-3194, DOI: 10.1002/mrm.26654 Retrieved from the Internet: URL:https://api.wiley.com/onlinelibrary/td m/v1/articles/10.1002%2Fmrm.26654> [retrieved on 2024-05-07] <br> * page 2, column 2 * | 1-15 | **TECHNICAL FIELDS SEARCHED (IPC)** <br><br> A61B <br> G01R |
| A | US 2014/018664 A1 (WEISS STEFFEN [DE] ET AL) 16 January 2014 (2014-01-16) <br> * paragraph [0121] * | 1-15 | |

-/--

The present search report has been drawn up for all claims

| Place of search | Date of completion of the search | Examiner |
|---|---|---|
| The Hague | 14 May 2024 | Costa Angeli, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 1 of 2

## EUROPEAN SEARCH REPORT

**Application Number**

EP 24 16 8931

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2003/220559 A1 (EHNHOLM GOSTA J [FI] ET AL) 27 November 2003 (2003-11-27)<br>* paragraph [0028] *<br>* claim 2 *<br>- - - - - | 1-15 | |
| A | US 2008/097189 A1 (DUMOULIN CHARLES [US] ET AL) 24 April 2008 (2008-04-24)<br>* figure 11 *<br>* paragraph [0052] *<br>- - - - - | 1-15 | |
| A | US 2014/039299 A1 (FOO THOMAS KWOK-FAH [US] ET AL) 6 February 2014 (2014-02-06)<br>* figure 7 *<br>* paragraph [0008] - paragraph [0009] *<br>- - - - - | 1-15 | |

**TECHNICAL FIELDS SEARCHED (IPC)**

The present search report has been drawn up for all claims

| Place of search | Date of completion of the search | Examiner |
|---|---|---|
| The Hague | 14 May 2024 | Costa Angeli, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 16 8931

This annex lists the patent family members relating to the patent documents cited in the above-mentioned European search report.
The members are as contained in the European Patent Office EDP file on
The European Patent Office is in no way liable for these particulars which are merely given for the purpose of information.

14-05-2024

| Patent document cited in search report | | | Publication date | Patent family member(s) | | | Publication date |
|---|---|---|---|---|---|---|---|
| EP 1745303 | B1 | | 27-09-2017 | CN | 1950712 | A | 18-04-2007 |
| | | | | EP | 1745303 | A1 | 24-01-2007 |
| | | | | JP | 5597342 | B2 | 01-10-2014 |
| | | | | JP | 2007534419 | A | 29-11-2007 |
| | | | | US | 2007164738 | A1 | 19-07-2007 |
| | | | | WO | 2005106518 | A1 | 10-11-2005 |
| EP 3140667 | B1 | | 23-12-2020 | CN | 106662626 | A | 10-05-2017 |
| | | | | EP | 3140667 | A1 | 15-03-2017 |
| | | | | JP | 6615122 | B2 | 04-12-2019 |
| | | | | JP | 6865802 | B2 | 28-04-2021 |
| | | | | JP | 2017517314 | A | 29-06-2017 |
| | | | | JP | 2020032218 | A | 05-03-2020 |
| | | | | US | 2017065830 | A1 | 09-03-2017 |
| | | | | WO | 2015169655 | A1 | 12-11-2015 |
| EP 2713868 | B1 | | 13-07-2016 | CN | 103547214 | A | 29-01-2014 |
| | | | | EP | 2713868 | A2 | 09-04-2014 |
| | | | | RU | 2013156839 | A | 27-06-2015 |
| | | | | US | 2014077811 | A1 | 20-03-2014 |
| | | | | WO | 2012160486 | A2 | 29-11-2012 |
| US 2010168553 | A1 | | 01-07-2010 | US | 2010168553 | A1 | 01-07-2010 |
| | | | | WO | 2006136029 | A1 | 28-12-2006 |
| US 2014018664 | A1 | | 16-01-2014 | CN | 103597369 | A | 19-02-2014 |
| | | | | EP | 2508907 | A1 | 10-10-2012 |
| | | | | EP | 2694992 | A1 | 12-02-2014 |
| | | | | JP | 6190357 | B2 | 30-08-2017 |
| | | | | JP | 2014509918 | A | 24-04-2014 |
| | | | | US | 2014018664 | A1 | 16-01-2014 |
| | | | | US | 2018160934 | A1 | 14-06-2018 |
| | | | | WO | 2012137148 | A1 | 11-10-2012 |
| US 2003220559 | A1 | | 27-11-2003 | AU | 2003237218 | A1 | 12-12-2003 |
| | | | | EP | 1509152 | A1 | 02-03-2005 |
| | | | | JP | 2005527292 | A | 15-09-2005 |
| | | | | US | 2003220559 | A1 | 27-11-2003 |
| | | | | WO | 03099154 | A1 | 04-12-2003 |
| US 2008097189 | A1 | | 24-04-2008 | NONE | | | |
| US 2014039299 | A1 | | 06-02-2014 | NONE | | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017129495 A1

**Non-patent literature cited in the description**

- **S. KOS**. MR-guided endovascular interventions: a comprehensive review on techniques and applications. *Eur. Radiol.*, April 2008, vol. 18 (4), 645-657